# EUROPEAN PATENT APPLICATION

(11) **EP 4 233 831 A2**
(43) Date of publication of application: **30.08.2023**
(21) Application number: 23173000.3
(22) Date of filing: 06.10.2020
(51) Int. Cl.: A61K 8/19

(54) **COMPOSITION FOR DELIVERING NITRIC OXIDE TO SKIN**

(30) Priority: 07.10.2019 GB 201914435; 07.10.2019 GB 201914437
(62) Divisional of application: 20793050.4
(71) Applicant: Insense Limited, Sharnbrook, Bedford MK44 1LQ (GB)
(72) Inventor: Jezek, Jan, Bedford, MK44 1LQ (GB); Davis, Paul, Bedford, MK44 1LQ (GB)
(74) Representative: Willett, Christopher David

(57) **Abstract**

A skin application composition comprising a first component, which is a porous water-absorbable material comprising S-nitrosothiol in dry inactive condition.

## Description

### Field of the Invention

The invention relates to a treatment composition, e.g. skin dressings, for application to a part of a human or animal body (for therapeutic or cosmetic purposes).

### Background and prior art

Nitric oxide is an essential signalling molecule in mammals and it is known to play a variety of roles, ranging from regulation of blood flow, neurotransmission and immune response. It is so important that a family of special enzymes, called nitric oxide synthases has evolved with the exclusive job of making controlled amounts of nitric oxide when and where it is needed, using the amino acid arginine as the precursor substance.

Nitric oxide is however a very hydrophobic compound and its solubility in water is therefore limited. Maximum solubility in water achievable under normal conditions is approximately 1.7 mM, with the solubility being similar to that of oxygen.

Nitric oxide also reacts rapidly with oxygen to form nitrogen dioxide. Nitrogen dioxide has no known role in maintaining or controlling homeostasis, or ability to respond to important stimuli in biological systems. In fact, nitrogen dioxide is known as a toxin and irritant.

In addition, the biology of mammals (and other vertebrates) is capable of safely managing the logistics of nitric oxide because of the abundance of thiol groups within the tissues, especially the skin. Nitric oxide spontaneously reacts with thiol groups (e.g. on proteins) to form S-nitrosothiol functional groups, in which form the nitric oxide can be safely and efficiently stored or transported. S-Nitrosothiols are compounds capable of releasing nitric oxide. Therefore nitric oxide can also be readily released on demand via the degradation of S-nitrosothiols.

Thus, mammalian biology deals with these problems of nitric oxide, when locked up as S-nitrosothiols it can't be oxidised to nitrogen dioxide and its insolubility in water is not a problem.

However, delivery of exogenous raw nitric oxide to a skin site can result in unacceptable losses to the production of nitrogen dioxide. Also, any nitric oxide that gets into the skin can become locked into keratin S-nitrosothiols in the wrong place, or the nitric oxide can't dissolve in the available water of the system being targeted.

Various strategies therefore need to be employed before the effective delivery of the valuable nitric oxide to a skin site can be considered.

US 6,103,275 discloses a biocompatible system for generating nitric oxide by bringing together a nitrite, a reductant and a particular acid. The nitrite and acid are typically kept separate until the moment of use.

As described, S-nitrosothiols can release free nitric oxide by spontaneous decomposition, and are therefore a very convenient delivery means for the reactive and insoluble nitric oxide. However, nitrosothiols spontaneously decompose and therefore have a limited lifetime as a delivery vehicle for nitric oxide.

WO 2006/095193 discloses a skin dressing in an inactive state, but can be made to become active and deliver S-nitrosothiols to a skin site. The dressing is kept inactive either by containing reactants for the nitrosothiols so that they form the nitrosothiols only immediately before use, or by keeping pre-formed nitrosothiols in a dry state, to be activated when needed by addition of water.

The rate of decomposition varies considerably depending on the side chain of the thiol. For example, whilst nitrosocysteine can be totally decomposed within minutes under normal conditions, it takes hours/days to achieve 100% decomposition of nitrosoglutathione. The decomposition is generally accelerated in the presence of Cu²⁺ and Hg²⁺.

### Summary of the Invention

The present invention mimics the natural biological process by directly synthesising S-nitrosothiols which can safely escort nitric oxide into the target location, without forming nitrogen dioxide, and in a water-soluble form. By their chemical nature, the S-nitrosothiols can readily exchange their nitric oxide with thiols of the body, thereby efficiently interposing the delivered nitric oxide into the body, harmonising with the nitric oxide logistics of the body.

Thus, the present invention provides a skin application composition comprising a first component comprising S-nitrosothiol in dry inactive condition.

As the S-nitrosothiols are in dry condition the treatment composition is in an inactive state. However, the treatment composition can be activated, by bringing an aqueous composition into contact with the dry S-nitrosothiol immediately prior to use, to allow the active delivery of the S-nitrosothiols to the skin site to be treated.

Dry condition means that there is no free water in the first component, such that no significant or measurable water loss occurs through evaporation under normal ambient conditions of temperature, pressure and humidity. Dry condition includes desiccated condition, which is an extra thoroughly dried condition. Desiccated condition means a condition maintained by storage in an environment enclosed by a moisture impermeable barrier, wherein the material is kept scrupulously free of water by means of an added desiccant.

Elevated nitric oxide can have beneficial effects on tissues suffering from inadequate blood perfusion, through its vasodilatory effect which causes blood capillaries in the vicinity to open up leading to improved blood circulation. The vasodilatory effect can also enhance transdermal delivery of materials such as a pharmaceutically active agent, e.g. hormones, analgesics etc, by accelerating delivery and uptake of the materials. The composition can thus also be used as an adjuvant for transdermal delivery, typically by having a composite dressing or patch, plaster, bandage, gauze etc. also including material for delivery.

Preferably the skin treatment composition is a skin dressing. The term "skin dressing" covers dressings such as patches, plasters, bandages, absorbent foams and gauze etc.. The term also includes material in amorphous or liquid form. The term covers dressings for application to body surfaces generally, including internal and external tissues, particularly the skin including the scalp.

Suitable S-nitrosothiols include S-nitrosoglutathione (preferably S-nitroso-L-glutathione, as this is the physiologically important version), S-nitrosocysteine, S-nitrosothioglycerol, S-nitroso-N-acetylcysteine, S-nitrosocaptopril, S-nitrosomercaptoethylamine, S-nitroso-3-mercaptopropanoic acid, S-nitroso-D-thioglucose and S-nitroso-N-acetyl-D, L-penicillamine. S-nitrosoglutathione is currently preferred, because of its relatively slow rate of decomposition to generate nitric oxide, resulting in satisfactory stability of the S-nitrosothiol in the dressing and consequential slow release of nitric oxide at an appropriate rate for skin benefits.

The S-nitrosothiol is typically prepared, e.g. by reaction between a nitrite and a thiol, prior to preparation of the treatment composition. This enables the S-nitrosothiols to be dried down to a suitable degree, to allow them to remain inactive and stable until required. This can be carried out by preparing an aqueous mix comprising a source of nitrite (such as a nitrite salt such as sodium nitrite or potassium nitrite) and a thiol (preferably thioglycerol, thioglucose or glutathione) followed by adjusting the pH of the mixture to less than 5.0, preferably less than 4.5, more preferably between 2.5 and 4.0. Any other additional ingredients can then be added (e.g. a chelating agent as discussed below) before removing water from the mix such as by freeze-drying or spray drying.

The or each treatment component may be in the form of a layer, e.g. in the form of a sheet, slab or film, that may produce from an amorphous material, not having any fixed form or shape, that can be deformed and shaped in three dimensions, including being squeezed through a nozzle.

Because the first component is in dry condition the S-nitrosothiol is in stable condition and available when required. When the S-nitrosothiol is wetted, e.g. by contact with a source of water, the S-nitrosothiol is solubilised and released. Sufficient water is required to form a contact liquid junction between the treatment composition and a water source.

The first component may be provided as a solid material with the dry S-nitrosothiol preferably dispersed in a reasonably homogeneous manner. The solid material preferably comprises a polymer material.

Preferred polymers include water-soluble polymers such as polyvinyl alcohol (PVA), polyvinyl pyrrolidone, cellulose or modified cellulose (such as carboxymethylcellulose). One preferred polymer material comprises PVA. PVA has convenient and acceptable properties for skin treatment use, e.g. being non-toxic. PVA is also easy to handle and use, readily forming a film on drying of a PVA solution in water, with the resulting film being easy to handle. PVA is also readily available and cheap. Cross-linking is not required to form a solid material, e.g. in the form of a film, although cross-linking may optionally be employed. PVA is available in a wide range of grades based on molecular weight and degree of hydrolysis, which affect the physical properties of the material. Appropriate grades of PVA can be readily selected to produce a polymer product having desired properties for a particular intended use. For example, for use in skin dressings, good results have been obtained by use of PVA with a molecular weight in the range 100,000 to 200,000, substantially fully hydrolysed (98-99% hydrolysed), e.g. in the form of code 36,316-2 from Aldrich, in non-cross-linked form, and M_{w} 31,000-50,000, 98-99% hydrolyzed - obtained from Sigma (363138).

Another suitable polymer material comprises polyvinylpyrrolidone (PVP). The properties of PVP are very similar to those of PVA, and PVP is also acceptable for skin treatment use. PVP is readily available in a range of different molecular weights. Appropriate grades of PVP can be readily selected. For example, good results have been obtained using a PVP having a molecular weight average of 360,000, e.g. in the form of code PVP360 from Sigma, in a non-crosslinked form.

Mixtures of polymer materials may be used.

The solid material is conveniently in the form of a sheet, layer or film, typically having a thickness in the range 0.01 to 1.0mm, preferably in the range 0.05 to 0.5mm.

The solid material may optionally include a support to provide rigidity when wet.

The solid material of the invention is conveniently made by mixing a solution of a polymer (e.g. an aqueous solution of PVA and/or PVP) and reagent, and drying the mixture to produce a solid material, e.g. forming film by a casting procedure. Suitable techniques are well known to those skilled in the art, and may be combined with the process described above.

The polymer material or materials are suitably used in appropriate amounts that result in formation of a film, with the upper limit of concentration typically being dictated by the limit of solubility (generally in water) and the lower limit of concentration being the point at which a film does not form. For PVA code 36,316-2 from Aldrich, the limit of solubility in water is about 6% w/w, resulting in a concentration of PVA in the film prior to drying of about 5%.

Such solid polymer materials are typically in dry condition, and therefore can be used on exuding wounds, which may provide the source of water. However, preferably such solid polymer materials will be provided with a second component comprising a source of water, as discussed below, and can therefore also be used on dry wounds.

Alternatively, the first component may be provided in the form of a porous water-absorbable material such as a mesh or foam, onto which the S-nitrosothiol is provided in dried form. Such a mesh or foam is preferably made from a solid water-absorbent polymer, e.g. silicone, polyethylene, polypropylene, polystyrene, polyurethane, polyacrylate and polyamide. Examples include the Allevyn^{™} range (Smith & Nephew), Biatain^{™} range (Coloplast), Lyofoam^{™} range (Molnlycke), Tielle^{™} range (Systagenix) and Tegaderm^{™} range (3M).

Alternatively the mesh or foam may be made from a material such as alginate. Such porous materials are applicable to exuding wounds. Examples include AlgisiteM^{™} range (Smith & Nephew), Biatain^{™} range (Coloplast), Kaltostat^{™} range (ConvaTec), Tegaderm^{™} range (3M) and Urgosorb^{™} range (Urgo).

Such meshes or foams may be prepared by applying an aqueous solution of S-nitrosothiol, e.g. by dipping, spraying etc., which is allowed to be absorbed into the porous structure of the mesh or foam. This is then followed by a drying step, leaving behind S-nitrosothiol in dry condition adhered to the structure of the mesh or foam.

If applied to an exuding wound, a mesh or foam can begin to absorb wound exudate to create a liquid flow pathway from the mesh or foam to the wound site. The S-nitrosothiol can then dissolve into the wound exudate and diffuse towards the wound site down the concentration gradient generated.

Alternatively, the first component may comprise a non-aqueous liquid matrix containing the S-nitrosothiol dispersed therein. The S-nitrosothiol may be dispersed in the matrix as a fine particulate material or may be dissolved into the matrix itself. Suitable non-aqueous liquids include propylene glycol, polyethylene glycol (e.g. PEG300, PEG400, PEG3350), and can include non-aqueous creams, ointments or lotions.

Such non-aqueous liquids are applicable to exuding wounds. In a similar manner to the meshes and foams, the water to enable release of the S-nitrosothiols may be provided by wound exudate.

Alternatively, the first component may be provided in the form of a particulate material such as a powder or in granular form. In such an arrangement the first component will generally include a carrier or bulking agent such as sugar or a polyol.

The first component may also comprise a chelating agent, capable of chelating divalent metal ions such as Cu²⁺, Zn²⁺ and/or Fe²⁺. It is known that such metal ions catalyse the decomposition of the S-nitrosothiol into nitric oxide, and therefore the chelating agent ensures that the S-nitrosothiol remains in an inactive state until desired for use. Suitable chelating agents include EDTA, EGTA, histidine and/or citrate.

The first component may also comprise a buffer, to ensure that the pH of any resulting solutions when brought into contact with water is in the range of from 4 to 7, preferably from 5 to 7.

The composition is activated, by bringing the first component into contact with a source of water (e.g. from a wound), resulting in release from the treatment composition of one or more S-nitrosothiols.

The S-nitrosothiol can therefore be released from the composition simply by applying the layer onto a moist surface (e.g. wound bed). Once released, S-nitrosothiol undergoes a slow decomposition to generate nitric oxide. The release of S-nitrosothiol from the layer can be relatively rapid. This can be demonstrated by applying the layer onto a moist skin, which results in rapid (within approximately 1 min) reddening of the skin due to dermal vasodilation. The reddening is totally reversible and disappears within several minutes after removal of the composition.

Alternatively the treatment composition may include a second component comprising a source of water (the aqueous component), separated from the S-nitrosothiol in dry condition.

The aqueous component may be a hydrated hydrogel. A hydrated hydrogel means one or more water-based or aqueous gels, in hydrated form. A hydrated hydrogel thus includes a source of water, for activation of the treatment composition. A hydrated hydrogel can also act to absorb water and other materials exuded from a wound site, enabling the treatment composition to perform a valuable and useful function by removing such materials from a wound site. The hydrated hydrogel also provides a source of moisture that can act in use to maintain a wound site moist, aiding healing.

Suitable hydrated hydrogels are disclosed in WO 03/090800. The hydrated hydrogel conveniently comprises hydrophilic polymer material. Suitable hydrophilic polymer materials include polyacrylates and methacrylates, e.g. as supplied by First Water Ltd in the form of proprietary hydrogels, including poly 2-acrylamido-2-methylpropane sulphonic acid (poly-AMPS) and/or salts thereof (e.g. as described in WO 01/96422), polysaccharides e.g. polysaccharide gums particularly xanthan gum (e.g. available under the Trade Mark Keltrol), various sugars, polycarboxylic acids (e.g. available under the Trade Mark Gantrez AN-169 BF from ISP Europe), poly(methyl vinyl ether co-maleic anhydride) (e.g. available under the Trade Mark Gantrez AN 139, having a molecular weight in the range 20,000 to 40,000), polyvinyl pyrrolidone (e.g. in the form of commercially available grades known as PVP K-30 and PVP K-90), polyethylene oxide (e.g. available under the Trade Mark Polyox WSR-301), polyvinyl alcohol (e.g. available under the Trade Mark Elvanol), cross-linked polyacrylic polymer (e.g. available under the Trade Mark Carbopol EZ-1), celluloses and modified celluloses including hydroxypropyl cellulose (e.g. available under the Trade Mark Klucel EEF), sodium carboxymethyl cellulose (e.g. available under the Trade Mark Cellulose Gum 7LF) and hydroxyethyl cellulose (e.g. available under the Trade Mark Natrosol 250 LR).

Mixtures of hydrophilic polymer materials may be used in a gel.

In a hydrated hydrogel of hydrophilic polymer material, the hydrophilic polymer material is desirably present at a concentration of at least 1%, preferably at least 2%, more preferably at least 5%, yet more preferably at least 10%, or at least 20%, desirably at least 25% and even more desirably at least 30% by weight based on the total weight of the gel. Even higher amounts, up to about 40% by weight based on the total weight of the gel, may be used.

Good results have been obtained with use of a hydrated hydrogel of poly-AMPS and/or salts thereof in an amount of about 30% by weight of the total weight of the gel.

By using a gel comprising a relatively high concentration (at least 2% by weight) of hydrophilic polymer material, the gel can function particularly effectively to take up water in use of the treatment composition, e.g. from serum exudates while in contact with a wound. Because the gel is an aqueous system, use of the treatment composition does not have the effect of inducing an overall dryness of the wound which would be undesirable. This is because water vapour pressure is maintained in the enclosed environment surrounding the skin in use of the treatment composition. The gel thus functions as an absorbent entity for the removal of moisture, e.g. wound exudate, that also provides a helpful background level of excess moisture.

The water-uptake capacity of a hydrated hydrogel, including a high concentration gel, enables the treatment composition to aid wound healing by removing substantial amounts of exudates, swelling-up as it does so. By using a carefully formulated, ready-hydrated gel, the wound is prevented from reaching a state of unhelpful dryness. Ready hydration also ensures the quick formation of an aqueous liquid interface between the treatment composition and the wound, thus preventing adhesion, which otherwise would interfere with easy lifting of the treatment composition when it has to be replaced. A good aqueous liquid interface between the wound and the treatment composition is also important in allowing any beneficial products carried in the gel to enter the wound through all of the available surface.

The hydrated hydrogel material is typically in the form of a solid layer, sheet or film of material that is typically cross-linked, and that may incorporate a mechanical reinforcing structure. The size and shape of the layer, sheet or film can be selected to suit the intended use of the treatment composition. Thicknesses in the range 0.05 to 5 mm, preferably 0.5 to 3 mm are particularly suitable. Examples of such gels include the ActiFormCool^{™} range (L&R) and Intrasite^{™} range (Smith & Nephew).

Alternatively, the hydrated hydrogel may be in the form of an amorphous gel not having a fixed form or shape that can be deformed and shaped in three dimensions, including being squeezed through a nozzle. Amorphous gels are typically not cross-linked or have low levels of cross-linking. A shear-thinning amorphous gel may be used. Such a gel is liquid when subjected to shear stress (e.g. when being poured or squeezed through a nozzle) but set when static. Thus the gel may be in the form of a pourable or squeezable component that may be dispensed, e.g. from a compressible tube or a syringe-like dispenser, comprising a piston and cylinder, typically with a nozzle of about 3 mm diameter. Such a gel may be applied in the form of a surface layer, or into a wound cavity as a fully conformable gel that fills the available space and contacts the wound surface. Examples of such gels include the Purilon^{™} range (Coloplast), Nu-Gel^{™} range (Systagenix), Granugel^{™} range (ConvaTec) and Intrasite^{™} range (Smith and Nephew).

A typical example of an amorphous gel formulation is: 15% w/w AMPS (sodium salt), 0.19% polyethylene glycol diacrylate and 0.01% hydroxycyclohexyl phenyl ketone, with the volume made up to 100% with analytical grade DI water. The reagents are thoroughly mixed and dissolved, then polymerised for between 30-60 seconds, using a UV-A lamp delivering approximately 100 mW/cm², to form the required hydrogel. This may be contained in plastic syringes from which the amorphous gel may then be dispensed from a syringe to a target site, as a surface layer or to fill a cavity.

The second component may alternatively comprise a hydro-cream carried in a suitable container such as a tub or a squeezable pouch or tube.

The second component may also comprise a buffer, to ensure that the pH of any resulting solutions is in the range of from 4 to 7, preferably from 5 to 7.

The second component may also comprise a source of divalent metal ions, such as Cu²⁺, Zn²⁺ and/or Fe²⁺. It is known that such metal ions catalyse the decomposition of the S-nitrosothiol into nitric oxide, and therefore providing them in the second composition can aid the delivery of nitric oxide when the first and second compositions are brought together.

When the first composition includes a chelating agent, it is preferred that the concentration of divalent metal ions in the second component exceeds the capacity of the chelating agent to chelate the divalent metal ions. This is so that, when brought together, any chelating agent in the first component will be capable of only chelating a fraction of the divalent metal ions in the second component. The excess of divalent metal ions can then act to catalyse the decomposition of the S-nitrosothiol to nitric oxide.

Thus, in one preferred embodiment the invention comprises a first component comprising a dry polymeric matrix, preferably dried PVA, containing the S-nitrosothiol and a second component comprising a layer of hydrated hydrogel. The second component may be used in contact with the skin, as the hydrated hydrogel has beneficial properties for skin contact, as discussed above, with the first component being placed on top of the second component. Provided the components are kept separate prior to use, the treatment composition remains in non-activated condition. However, when the two components are brought into contact, this has the effect of activating the treatment composition.

In one preferred embodiment of this type, the treatment composition comprises a layer of dried PVA containing pre-generated S-nitrosothiol. The layer is formed from PVA, a source of nitrite (preferably potassium nitrite) and a thiol (preferably L-glutathione). In a typical example of this embodiment both of these additives are added to the PVA solution prior to drying. S-nitrosothiol (GSNO in the preferred case) is generated within the layer during the drying step. Nitrosothiols are known to be rather unstable in aqueous solutions. Nevertheless, GSNO was found very stable in the dried layer of PVA, especially if stored in a moisture-free atmosphere.

In another preferred embodiment, the treatment composition comprises components which are amorphous. This is particularly the case for the preferred embodiment where the S-nitrosothiol is dispersed in a non-aqueous liquid matrix. The amorphous components can be in the form of e.g. a gel, semi-solid, paste, cream, lotion or liquid. Such an amorphous component may be provided on its own and derive the needed water from the wound exudate itself. Alternatively water may be provided by hydrated hydrogels or other amorphous material, as discussed above.

The two amorphous components are kept separate until it is desired to apply the treatment composition to a body surface. Conveniently they are packaged in a container having a nozzle, through which the amorphous components can be delivered. Preferably, the two components are packaged in a two compartment dispenser, preferably being operable to deliver both components simultaneously.

Another preferred embodiment is the use of a mesh or foam comprising the S-nitrosothiol in dried condition. Such foams being water-absorbable can be used on exuding wounds to derive the water from the wound. Alternatively such foams may be provided with a source of water as discussed above, or wetted immediately prior to use.

The treatment composition optionally includes, or is used with, a covering or outer layer for adhering a dressing to the skin of a human or animal in known manner.

Treatment compositions in accordance with the invention can be manufactured in a range of different sizes and shapes for treatment of areas of skin e.g. wounds of different sizes and shapes. Appropriate amounts of reagents for a particular dressing can be readily determined by experiment.

Treatment composition components are suitably stored prior to use in sterile, sealed, water-impervious packages, e.g. laminated aluminium foil packages. To ensure a dry condition is maintained, desiccant material is desirably included in the packages.

In use, the treatment composition component or components are removed from their packaging and located in appropriate order on the skin of a human or animal, e.g. over a wound or other region of skin to be treated for cosmetic or therapeutic purposes. The treatment composition may also be used as an adjuvant for transdermal delivery, as noted above.

### Examples

A number of model systems were prepared to demonstrate the release rate of S-nitrosothiols. The systems are detailed in tables at the start of each results section below. For each system, sample aliquots from the aqueous component were taken, at three time points after the system 'activation' (i.e. all components being brought together) to confirm the presence of S-nitrosothiol release. The first time-point was always t=zero (i.e. measuring S-nitrosothiol in the aqueous component prior to being 'activated' with the dry/non-aqueous component to demonstrate there was no S-nitrosothiol at the start, which was the case in all systems), then t=2 hours after activation and finally t=6 hours after activation.

### Materials

- Sodium nitrite (300 mM) in DI water
- Glutathione (300 mM) in DI water
- Lactic acid (100 mM) in DI water - (adjusted to pH 4.0 with 0.2 M NaOH)
- Sorbitol (1 M) in DI water
- Polyvinyl alcohol (7.5% w/w) in DI water - M_{w} 31,000-50,000, 98-99% hydrolyzed - obtained from Sigma (363138)
- EDTA (disodium) (5 mM) in DI water
- Copper (2+) Nitrite (5 mM) in DI water

### PVA Stock Solution Manufacture Procedure

462.5 ml of DI water was measured out and heated on hot plate to constant temperature between 80-85°C - controlled with digital thermometer. 37.5 g PVA powder was measured out and divided onto 5 × 7.5g aliquots. Single aliquots of the PVA powder were added to the heated water which was being stirred (preventing PVA coagulation). Throughout the additions, the water/PVA temperature was maintained at 80-85°C. Additions were repeated while maintaining the temperature of the water/PVA mix until the PVA is dissolved. After removal from the hotplate and cooling, the final volume was made up to 500 ml with DI water.

### PVA Films Manufacture

PVA films were produced by mixing the PVA stock solution with active components and allowing the mixture to dry in a Petri plate at 40°C. 20 ml of each pre-prepared PVA solution comprising the active components was poured into 10 × 10 cm Petri plates and left to dry overnight in an incubator at 40°C. The composition of the PVA mixtures prior to drying is shown in Table 1. Glutathione and nitrite were allowed to react together to form S-nitrosothiol prior to formation of the PVA film.

The PVA films comprising S-nitrosothiol were subsequently used to follow the rate of S-nitrosothiol release after being brought in contact with an aqueous system.

**Table 1**

| **Film ID** | **Film Components** |
|---|---|
| PVA4 | PVA (5% w/w) |
| | Glutathione (30 mM) |
| | Sodium nitrite (30 mM) |
| | Lactic acid (5 mM) |
| | at pH 4.0 |
| PVA5 | PVA (5% w/w) |
| | Glutathione (30 mM) |
| | Sodium nitrite (30 mM) |
| | Lactic acid (5 mM) |
| | EDTA (0.05 mM) |
| | at pH 4.0 |

### Powder Manufacture

Powders comprising an S-nitrosothiol were produced by mixing glutathione and sodium nitrite in aqueous sorbitol background, allowing the glutathione and sodium nitrite to react and form S-nitrosothiol, followed by drying the mixture. For each powder, 20 ml of each pre-prepared solution was poured into 10 × 10 cm Petri plates and left to dehydrate for 24 hours in an incubator at 40°C, followed by a thorough desiccation. Once in powder form, the formulations were dispersed in neat Propylene Glycol (0.1 g of powder to 1ml Propylene Glycol).

The composition of the aqueous mixtures for powder preparation prior to drying is shown in Table 2.

The powders comprising S-nitrosothiol, suspended in non-aqueous Propylene Glycol were subsequently used to follow the rate of S-nitrosothiol release after being brought in contact with an aqueous system.

**Table 2**

| **Powder ID** | **Powder Components** |
|---|---|
| P3 | Sorbitol (500 mM) |
| | Glutathione (30 mM) |
| | Sodium nitrite (30 mM) |
| | Lactic acid (5 mM) |
| | at pH 4.0 |
| P4 | Sorbitol (500 mM) |
| | Glutathione (30 mM) |
| | Sodium nitrite (30 mM) |
| | Lactic acid (5 mM) |
| | EDTA (0.05 mM) |
| | at pH 4.0 |

### Impregnated Foams Manufacture

Impregnated foams comprising S-nitrosothiol were produced by allowing the foam to absorb an aqueous solution comprising glutathione and sodium nitrite, allowing the glutathione and sodium nitrite to react and form S-nitrosothiol, followed by thorough drying at 40°C.

ActivHeal^{™} (Advanced Medical Solutions) foam was used.

The composition of the aqueous mixtures for impregnated foam preparation prior to drying is shown in Table 3.

The impregnated foams comprising S-nitrosothiol were subsequently used to follow the rate of S-nitrosothiol release after being brought in contact with an aqueous system.

**Table 3**

| **Foam ID** | **Impregnated Foam Components** |
|---|---|
| F1 | Glutathione (30 mM) |
| | Sodium nitrite (30 mM) |
| | Lactic acid (5 mM) |
| | at pH 4.0 |
| F2 | Glutathione (30 mM) |
| | Sodium nitrite (30 mM) |
| | Lactic acid (5 mM) |
| | EDTA (0.05 mM) |
| | at pH 4.0 |

For impregnated foams production, 20mls of each pre-prepared component solution was poured into 10 × 10cm Petri plates. 4 × 4 cm² pieces of the foam dressing were placed into the solution and the solution allowed to soak into the dressings over 4 hours. The foam dressing pieces were then removed and dried overnight in an incubator at 40°C.

### Aqueous Components

The pre-prepared PVA films/Powders/Impregnated Foams require contact with an aqueous component to activate S-nitrosothiol generation. The aqueous components used are shown in Table 4.

**Table 4**

| **AQ ID** | **Aqueous Component** |
|---|---|
| AQ1 | Sheet hydrogel |
| AQ2 | Sheet hydrogel imbibed with 5mM Copper Nitrite Solution (50µL of Cu(NOa)z per 1cm² hydrogel) |
| AQ3 | Amorphous Hydrogel |
| AQ4 | Amorphous Hydrogel (50µL of 5mM Cu(NO₃)₂ per 1cm2 hydrogel) |
| AQ5 | DI water |
| AQ6 | 0.2mM Cu(NO₃)₂ Solution (in DI water) |

Details of the sheet hydrogel and amorphous hydrogel materials are shown in Table 5.

**Table 5**

| **Component** | **Name** | **Manufacturer** | **Details** |
|---|---|---|---|
| Sheet hydrogel | ActiformCool | Activa | 70% H₂O: 30% Acrylic Polymer (Taurate derivative). |
| | | | Phenoxyethanol as preservative. Used in moderate to heavily exuding wounds |
| Amorphous Hydrogel | ActivHeal | Advanced Medical Solutions | Hydrogel with high water content - 85%. Used in nil to low exudate wounds |

### S-Nitrosothiol Measurement

The presence of S-nitrosothiols was measured by an Absorbance reading at 490nm using the Griess reagent method described below. S-nitrosothiol concentration can be calculated from the absorbance measurement using the extinction coefficient of ca. 10,000 M⁻¹ cm⁻¹. Absorbance measurement was carried out using Fisherbrand^{™} Digital Colorimeter Model 45.

Two different methods were required to measure S-nitrosothiol concentration depending on whether a Hydrogel (AQ1 to AQ4) or a Solution (AQ5 and AQ6) were utilised as the aqueous component.

### Reagents for S-nitrosothiol measurement

| | |
|---|---|
| Reagent 1: | Na-phosphate buffer (pH 7.4, 0.1 M). |
| Reagent 2: | Griess reagent: 20 mg of N-(1-Naphthyl)ethylendiamine dihydrochloride (NADD) + 500 mg of sulphanilamide dissolved in 2 mL of DMSO. |
| Reagent 3: | Mercuric chloride (10 mM) in DMSO (13.58 mg of HgCl2 in 5 mL of DMSO). |

### Procedure to measure S-nitrosothiol concentration in gels

1. Dispense 25 mL of Reagent 1 and 825 µL of Reagent 2 into a 250 ml pot
2. Weigh accurately 300 mg of the hydrogel and immerse it in the reagent mix. Incubate while shaking mildly for 30 min.
3. Transfer 2.6 ml of the reagent mix from the pot into a plastic cuvette
4. Add 25 µl of Reagent 3
5. Read absorbance of the resulting mixture at 490 nm in 10 min

### Procedure to measure S-nitrosothiol concentration in solutions

1. Dispense 1.5 mL of Reagent 1 into a plastic cuvette
2. Add 200 µL of the sample
3. Add 1.17 µL of DI water
4. Add 100 µL of Reagent 2
5. Add 30 µL of Reagent 3 and mix thoroughly
6. Read absorbance of the resulting mixture at 490 nm in 10 min

### Results

### S-nitrosothiol measurements in PVA film systems

Release of S-nitrosothiol from PVA film systems following activation by contact with aqueous systems is shown in Table 6. In each case the measurements were repeated, and the results are shown in mAU.

**Table 6**

| **PVA film** | **Aqueous component** | **Absorbance at t=0** | **Absorbance at 2 hours** | **Absorbance at 6 hours** |
|---|---|---|---|---|
| PVA4 | Aq1 | 0 | 10 | 60 |
| | | 0 | 10 | 50 |
| PVA4 | Aq2 | 0 | 0 | 60 |
| | | 0 | 0 | 40 |
| PVA4 | Aq3 | 0 | 70 | 90 |
| | | 0 | 70 | 80 |
| PVA4 | Aq4 | 0 | 20 | 40 |
| | | 0 | 20 | 50 |
| PVA5 | Aq1 | 0 | 10 | 20 |
| | | 0 | 0 | 20 |
| PVA5 | Aq2 | 0 | 20 | 30 |
| | | 0 | 10 | 30 |
| PVA5 | Aq3 | 0 | 40 | 80 |
| | | 0 | 40 | 90 |
| PVA5 | Aq4 | 0 | 20 | 100 |
| | | 0 | 20 | 90 |

### S-nitrosothiol measurements in foam based systems

Release of S-nitrosothiol from foam based systems following activation by contact with aqueous systems is shown in Table 7. In each case the measurements were repeated, and the results are shown in mAU.

**Table 7**

| **Foam component** | **Aqueous component** | **Absorbance at t=0** | **Absorbance at 2 hrs** | **Absorbance at 6 hours** |
|---|---|---|---|---|
| Foam 1 | Aq1 | 0 | 30 | 90 |
| | | 0 | 20 | 90 |
| Foam 1 | Aq2 | 0 | 20 | 40 |
| | | 0 | 20 | 40 |
| Foam 1 | Aq3 | 0 | 70 | 90 |
| | | 0 | 60 | 80 |
| Foam 1 | Aq4 | 0 | 70 | 80 |
| | | 0 | 70 | 90 |
| Foam 1 | Aq5 | 0 | 820 | 750 |
| | | 0 | 920 | 810 |
| Foam 1 | Aq6 | 0 | 370 | 440 |
| | | 0 | 360 | 390 |
| Foam 2 | Aq1 | 0 | 0 | 0 |
| | | 0 | 0 | 0.00 |
| Foam 2 | Aq2 | 0 | 30 | 20 |
| | | 0 | 40 | 20 |
| Foam 2 | Aq3 | 0 | 190 | 230 |
| | | 0 | 160 | 210 |
| Foam 2 | Aq4 | 0 | 170 | 230 |
| | | 0 | 180 | 250 |
| Foam 2 | Aq5 | 0 | 670 | 920 |
| | | 0 | 500 | 740 |
| Foam 2 | Aq6 | 0 | 810 | 690 |
| | | 0 | 820 | 750 |

### S-nitrosothiol measurements in systems based on a non-aqueous liquid component

Release of S-nitrosothiol from powder based non-aqueous systems following activation by contact with aqueous systems is shown in Table 8. In each case the measurements were repeated, and the results are shown in mAU.

**Table 8**

| **Non-aqueous component 1** | **Aqueous component** | **Absorbance at t=0** | **Absorbance at 2 hrs** | **Absorbance at 6 hours** |
|---|---|---|---|---|
| P3 | Aq3 | 0 | 30 | 50 |
| | | 0 | 30 | 50 |
| P3 | Aq4 | 0 | 40 | 30 |
| | | 0 | 30 | 30 |
| P3 | Aq5 | 0 | 80 | 50 |
| | | 0 | 80 | 50 |
| P3 | Aq6 | 0 | 70 | 0 |
| | | 0 | 70 | 0 |
| P4 | Aq3 | 0 | 10 | 40 |
| | | 0 | 0 | 30 |
| P4 | Aq4 | 0 | 0 | 0 |
| | | 0 | 0 | 0 |
| P4 | Aq5 | 0 | 80 | 60 |
| | | 0 | 70 | 60 |
| P4 | Aq6 | 0 | 50 | 0 |
| | | 0 | 30 | 0 |

### Conclusions

Release of S-nitrosothiol from non-aqueous components based on PVA films, a foam based systems and powder based systems was demonstrated on contact with an aqueous component.

## Claims

1. A skin application composition comprising a first component, which is a porous water-absorbable material comprising S-nitrosothiol in dry inactive condition.

2. A skin application composition according to claim 1, wherein the application composition is a skin dressing.

3. A skin application composition according to claim 1 or claim 2, wherein the porous water-absorbable material is a mesh or foam.

4. A skin application composition according to claim 3, wherein the mesh or foam is made from a solid water-absorbent polymer.

5. A skin application composition according to claim 3, wherein the mesh or foam is made from alginate.

6. A skin application composition according to any one of the preceding claims, wherein the first component comprises a chelating agent capable of chelating divalent metal ions.

7. A skin application composition according to any one of the preceding clams, in combination with wound exudate.

8. A skin application composition according to any one of the preceding claims, which comprises a second component comprising a source of water.

9. A skin application composition according to claim 8, wherein the second component comprises a source of divalent metal ions.

10. A skin application composition according to claim 7, 8 and 9, wherein the concentration of divalent metal ions exceeds the capacity of the chelating agent to chelate divalent metal ions.

11. A method of preparation of a skin application composition according to any one of claims 3 to 10, applying an aqueous solution of S-nitrosothiol so that it is absorbed into the porous structure of the mesh or foam, followed by a drying step, leaving behind S-nitrosothiol in dry condition adhered to the structure of the mesh or foam.
